# EUROPEAN PATENT APPLICATION

(11) **EP 2 140 810 A1**
(43) Date of publication of application: **06.01.2010**
(21) Application number: 09075224.7
(22) Date of filing: 29.10.2003
(51) Int. Cl.: A61B 5/15

(54) **A device for use in an improved method of lancing skin for the extraction of blood**

(30) Priority: 30.10.2002 US 422228 P
(62) Divisional of application: 03777996.4
(71) Applicant: LifeScan, Inc., Milpitas, CA 95035-6312 (US)
(72) Inventor: Allen, John J., Mendota Heights, MN 55118 (US)
(74) Representative: Mercer, Christopher Paul

(57) **Abstract**

The present invention relates to a device for use in a method of lancing skin through an outer surface of said skin to obtain a sample of blood, the device comprising a lancing instrument having a sharpened end and a channel extending from adjacent said sharpened end to a sensor attached to a proximal end of said lancing instrument. The sharpened tip of the lancing element may be forced into said skin to a first predetermined depth below said outer surface, wherein said sharpened tip creates an incision in said skin surface; then completely withdrawn from the incision. Blood may be drawn through said channel to the sensor.

## Description

### BACKGROUND OF THE INVENTION

### Field of the Invention

The present invention relates, in general, to a device for use in an improved method of lancing skin and, more particularly, in an improved method wherein the lancing element is removed after creating an incision and then reinserted into the incision at a lesser depth of penetration.

### Background of the Invention

In in-situ testing of blood glucose, a glucose meter is placed against the skin and blood is sampled and measured without moving the meter. In one method of in-situ testing, a glucose sensor strip is combined with a lancing element positioned at a distal end thereof, the glucose sensor strip is then positioned in a meter adapted to launch the strip and lancing element combination toward the skin where the lancing element forms an incision. Blood or other bodily fluids such as, for example, interstitial fluid, may then be extracted from the incision and moved to the glucose sensor strip where it can be measured using, for example, an electrochemical process.

When lancing skin using an in-situ test strip, it is desirable to ensure that blood be transferred efficiently from the incision to the test strip, using as little blood as possible. Efficient transfer of blood from the incision means that more of the blood is actually used to test for analyte (e.g. glucose) levels, reducing the total blood required and, therefore, the incision size required for the test. Smaller incisions are particularly desirable because, in general, it is desirable to reduce the pain experienced by the user. Further, smaller incisions generally heal faster and are not as likely to re-open once healed.

Thus, when using an in-situ test, it is desirable to create an incision which is very small while maximizing the amount of blood generated by that incision. A number of factors influence the amount of blood generated by a particular incision. Many of those factors cannot be controlled. One of the factors which reduces the amount of blood available at a particular incision is the tendency of the incision to close and reseal after the lancing element is removed. Another factor which reduces the amount of blood available at a particular incision is the tendency of the wound to seal around the lancing element if the lancing element is left in the wound.

It would, therefore, be advantageous to develop a method of lancing which increases the amount of blood available for testing at a particular incision site. It would further be advantageous to develop a method of lancing which increases the amount of blood available for lancing by preventing the wound from resealing during the testing process. It would further be advantageous to develop a method of lancing which increases the amount of blood available for lancing by preventing the wound from sealing around the lancing element during the testing process.

### SUMMARY OF THE INVENTION

The present invention provides a lancing instrument for lancing skin. The lancing instrument has proximal and distal ends and comprises a lancing element having a sharpened end; a fluid channel extending from the sharpened end; and a channel tip integrated with the lancing element at the distal end and offset in the proximal direction from the sharpened end of the lancing element. The lancing element is adapted for insertion to form an incision. The channel tip is adapted to increase the separation of the incision. The lacing element and said channel tip are adapted to be maintained in said incision while bodily fluids are drawn into the fluid channel.

In a method for using a lance, as set forth above, a lancing tip is first inserted and then is retracted completely out of the lance wound site. The lancing tip is then re-inserted into the same wound to a depth which is shallower than the original penetration. This method of lancing facilitates the expression of blood and its seamless transfer into a test strip integrated with a lance.

In a method for using a lance, as set forth above, the lancing element is inserted through an outer surface of the skin to obtain a sample of blood. In one embodiment
, the method includes using a lancing instrument having a sharpened end and a fluid channel extending from the sharpened end to a sensor attached to a proximal end of the lancing instrument, forcing the sharpened tip of the lancing element into the skin to a first predetermined depth, wherein the sharpened tip creates an incision in the skin surface and a wound below the skin surface and completely withdrawing the lancing element, including the sharpened tip. After completely withdrawing the sharpened tip from the incision, sharpened tip is forced back into the incision to a second predetermined depth, wherein the second predetermined depth is not as deep as the first predetermined depth. Once the sharpened tip is positioned at the second predetermined depth, blood is drawn through the channel to the sensor.

In a method for using a lance, as set forth above,
the method may further include ensuring that the second predetermined depth is sufficient to ensure that a distal end of the channel is positioned below the surface of the skin. For example, in one embodiment, the method may include setting the first predetermined depth in the range of approximately 0.25 to 1.5 mm. Further, in one embodiment , the method may include setting the second predetermined depth is in the range of approximately 0.05 to 0.25mm.

In a method for using a lance, as set forth above, the lancing element is inserted through an outer surface of the skin to obtain a sample of blood. In one embodiment
, the method includes providing pressure on the skin in a region surrounding the incision site (i.e. the site where the incision is to be matie). Then using a lancing instrument having a sharpened end and a fluid channel extending from the sharpened end to a sensor attached to a proximal end of the lancing instrument, forcing the sharpened tip of the lancing element into the skin to a first predetermined depth, wherein the sharpened tip creates an incision in the skin surface and a wound below the skin surface and completely withdrawing the lancing element, including the sharpened tip. After completely withdrawing the sharpened tip from the incision, sharpened tip is forced back into the incision to a second predetermined depth, wherein the second predetermined depth is not as deep as the first predetermined depth. Once the sharpened tip is positioned at the second predetermined depth, blood is drawn through the channel to the sensor.

In a method for using a lance, as set forth above, a lancing tip is extended beyond an opening at a distal end of a lancing device, such as, for example, an in-situ meter. The lancing tip is extended to a first extension length beyond the opening to create a wound and then retracted past the opening. The lancing tip is then re-extended past the opening to a second extension length which is less than the first extension length and blood is drawn up the lancing element from the wound into a test strip integrated with a lance.

In a method for using a lance, as set forth above, the lancing element is extended a first distance past an opening and through an outer surface of the skin to obtain a sample of blood. In one embodiment , the method includes using a lancing instrument having a sharpened end and a fluid channel extending from the sharpened end to a sensor attached to a proximal end of the lancing instrument, extending the sharpened tip a first extension length beyond an opening, forcing the sharpened tip of the lancing element into the skin, wherein the sharpened tip creates an incision in the skin surface and a wound below the skin surface, withdrawing the lancing element into the opening and extending the sharpened tip a second extension length beyond the opening, wherein the second extenstion depth is less than the first extension depth. Once the sharpened tip is positioned at the second extension depth, blood is drawn through the channel to the sensor.

In a method for using a lance, as set forth above,
the method may further include ensuring that the extension length is sufficient to ensure that a distal end of the channel is positioned below the surface of the skin. For example, in one embodiment, the method may include setting the first extension length in the range of approximately 0.25 to 1.5 mm. Further, in one embodiment
, the method may include setting the second extension length is in the range of approximately 0.05 to 0.25mm.

In a method for using a lance, as set forth above, the lancing element is inserted through an outer surface of the skin to obtain a sample of blood In one embodiment
, the method includes providing pressure on the skin in a region surrounding the incision site (i.e. the site where the incision is to be made). Then using a lancing instrument having a sharpened end and a fluid channel extending from the sharpened end to a sensor attached to a proximal end of the lancing instrument, forcing the sharpened tip of the lancing element though an opening into the skin to a first extension length beyond the opening, wherein the sharpened tip creates an incision in the skin surface and a wound below the skin surface and completely withdrawing the lancing element, including the sharpened tip into the opening. After completely withdrawing the sharpened tip into the opening, extending the sharpened tip through the opening and into the incision to a second extension length beyond the opening, wherein the second extension length is not as long as the first extension length. Once the sharpened tip is positioned at the second extension length, blood is drawn through the channel to the sensor.

In a method for using a lance, as set forth above,
the method may further include providing a milking ring wherein the pressure in the region surrounding the incision site is exerted by the milking ring. The milking ring is positioned on the skin prior to the step of forcing the sharpened tip into the skin and may be maintained throughout the remainder of the procedure. In this embodiment the milking ring provides a pressure sufficient to facilitate the flow of bodily fluids into the channel after the reinsertion of the lancing tip into the wound. In one embodiment
, the milking ring provides a pressure in a range of approximately 0.5 to 1.5 pounds. In a further embodiment , the method may include positioning the milking ring against the skin for a predetermined period of time prior to launching the lancing element. In a further embodiment , the predetermined period of time may be three seconds or more.

### BRIEF DESCRIPTION OF THE DRAWINGS

The novel features of the invention are set forth with particularity in the appended claims. A better understanding of the features and advantages of the present invention will be obtained by reference to the following detailed description that sets forth illustrative embodiments, in which the principles of the invention are utilized, and the accompanying drawings of which:
Figure 1 is a perspective view of a lancing element and strip according to the present invention.
Figure 2 is a perspective view of the top layer of a lancing element.
Figure 3A is a perspective view of a lancing element tip immediately before penetration of the surface of the skin.
Figure 3B is a side view of the lancing element illustrated in Figure 3A.
Figure 3C is a perspective view of a lancing element as the tip of the lancing element begins to penetrate the surface of the skin.
Figure 3D is a side view of the lancing element as the tip of the lancing element begins to penetrate the surface of the skin.
Figure 3E is a perspective view of the lancing element as it reaches its full depth of penetration beneath the surface of the skin.
Figure 3F is a side view of the lancing element as it reaches its full depth of penetration beneath the surface of the skin.
Fig 3G is a perspective view of the lancing element after it is fully withdrawn from the skin.
Fig 3H is a side view of the lancing element after it is full withdrawn from the skin.
Figure 3I is a perspective view of the lancing element as it re-enters the surface of the skin through the incision created during the first entry.
Figure 3J is a side view of the lancing element as it re-enters the surface of the skin through the incision created during the first entry.
Figure 3K is a perspective view showing the lancing element being used to draw blood from a forearm.

### DETAILED DESCRIPTION OF ILLUSTRATIVE EMBODIMENTS OF THE INVENTION

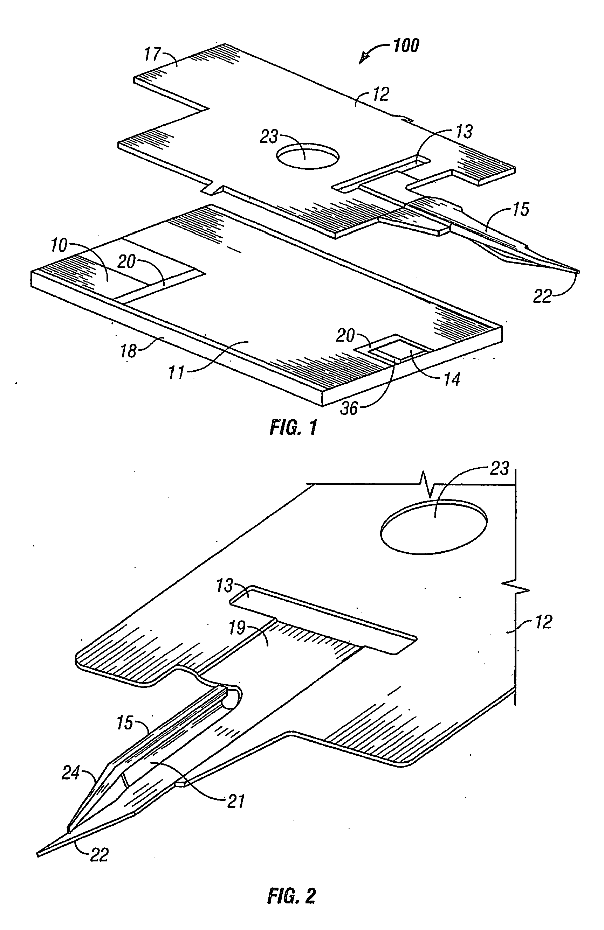

Figure 1 is a perspective view of a lancing element and strip according to the present invention. In Figure 1, lancing element 15 is connected to sensor strip 100. Sensor strip 100 may be, for example, a glucose sensor strip which uses electrochemistry to measure the amount of glucose in a bodily fluid, such as, for example, blood or interstitial fluid. In Figure 1, lancing element further includes lancing tip 22. Sensor strip 100 further includes first electrode contact 10, adhesive layer 11, conductive substrate 12, vent hole 13, analyte sensing layer 14, second electrode contact 17, insulating substrate 18, insulating layer 20, registration hole 23 and working electrode 36.
Figure 2 is a perspective view of the top layer of a lancing element and strip according to the present invention. In Figure 2 top layer is formed of conductive substrate 12. In the embodiment illustrated in Figure 2, conductive substrate 12 includes vent hole 13 and registration hole 23. In Figure 2, lancing element includes lancing tip 22, channel tip 24 and fill channel 21.

One embodiment of a lancing element and sensor strip suitable according to the present invention may be described with reference to Figures 1 and 2. In the embodiment illustrated in Figures 1 and 2, sensor strip 100 includes first electrode contact 10, wherein first electrode contact may be screen printed on an insulating substrate 18, and a second electrode contact 17, wherein said second electrode contact comprises a portion of conductive substrate 12 which is contiguous with top reference electrode 19 and lancing element 15.

In the embodiment of the lancing element and sensor strip illustrated in Figures 1 and 2, the orientation of said first electrode contact 10 and second electrode contact 17 are arranged such that an analyte measurement meter, such as, for example, a glucose meter (not shown) can establish electrical contact with sensor strip 100. In the illustrated embodiment, the electrodes are arranged on the same side of insulating substrate 18 to facilitate contact of both electrodes at the proximal end of sensor strip 100.

Sensor strip 100 is manufactured using adhesive layer 11 to attach insulating substrate 18 to conductive substrate 12. Adhesive layer 11 could be implemented in a number of ways, including using pressure sensitive material, heat activated material, or UV cured double sided adhesive material. Conductive substrate 12 may be, for example, a conductive substrate that is a sheet of electrically conductive material such as gold or plated stainless steel. The geometry of conductive substrate 12 may be formed by, for example, stamping process or photo-etching. In the embodiment illustrated in Figures 1 and 2, lancing element 15 may be manufactured as an integral part of conductive substrate 12. Vent hole 13, may be formed by, for example, punching through conductive layer 12. Vent hole 13 is used to facilitate the transport of bodily fluid up lancing element 15 and across analyte sensing layer 14. Registration hole 23 may be formed during the stamping process of making conductive substrate 12.

In one embodiment of the invention, analyte sensing layer 14 may be, for example, a glucose sensing layer, including an enzyme, a buffer, and a redox mediator. Analyte sensing layer 14 may preferably be deposited on top of working electrode 36. Where analyte sensing layer 14 is used to detect the presence and concentration of glucose in a bodily fluid, at least a portion of glucose sensing layer 14 dissolves in the bodily fluid and is used to convert the glucose concentration into an electrically measured parameter which is proportional to the glucose concentration in the sample.

In the embodiment illustrated in Figures 1 and 2, lancing element 15 has a proximal and distal end and the proximal end is integrated with the top reference electrode 19 and said distal end is integrated with a lancing tip 22 and channel tip 24. The lancing element is formed by the process of stamping or photo-etching a conductive material sheet and bending it to the geometry shown in Figure 2. In one embodiment, lacing tip 22 and channel tip 24 are slightly offset by about 0.005 to 0.020", the design of lancing element 15 is adapted to assist in improving skin separation. The geometry illustrated in Figures 1 and 2 may enhance fluid egress because it helps spread and hold open a skin wound. In the embodiment illustrated in Figures 1 and 2, the lancing element 15 is contiguous with the top reference electrode 19 and electrode contact 17.

In the embodiment of the invention illustrated in Figure 2, lancing element 15 includes fill channel 21, wherein capillary fill channel 21 facilitates the flow of body fluid from the wound to the analyte sensing layer 14. Fill channel 21 may facilitate the flow of bodily fluids by, for example, wicking or capillary action. In the embodiment illustrated in Figures 1 and 2 fill channel 21 has an open geometry which facilitates the wicking of viscous samples and provides for simpler manufacturing techniques when compared with closed capillary channels.

In the embodiment of sensor strip 100 illustrated in Figure 1, insulating substrate 18 consists of material such as polyester or ceramic on which a conductive material can be printed onto the insulating layer through silk-screening, sputtering, or electro-less deposition. Conductive material deposited on insulating substrate 18 forms first electrode contact 10 and working electrode 36. Insulating layer 20 may be, for example, screen printed to form a boundary for the electrode contact 10 and the bottom working electrode.

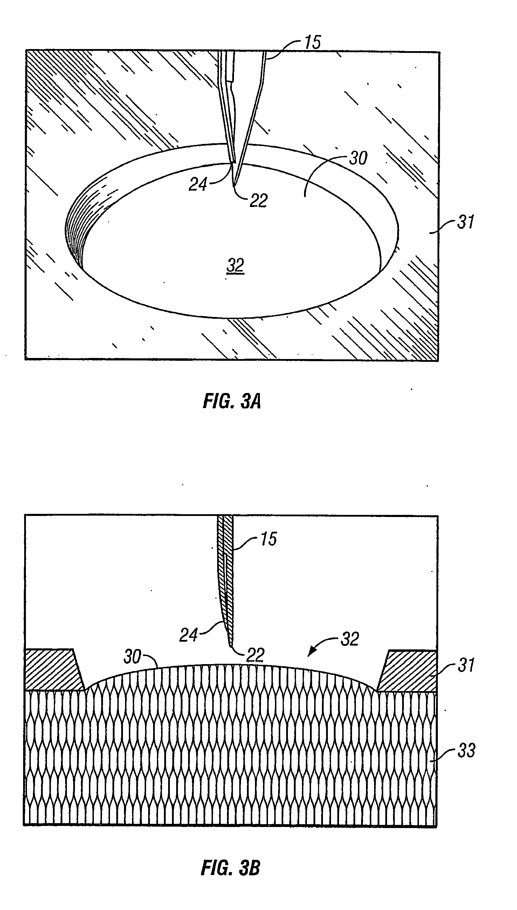

Figure 3A is a perspective view of a lancing element tip immediately before penetration of the surface of the skin. More particularly, Figure 3A is a perspective view of a lance 15 before lancing tip 22 penetrates skin surface 30. Figure 3B is a side view of the lancing element illustrated in Figure 3A. More particularly Figure 3B is side view of lancing element 15 before lancing tip 22 penetrates skin surface 30. In Figures 3A and 3B, milking ring 31 is placed against skin surface 30, causing skin surface 30 to bulge into milking opening 32. Opening 32 may further be the opening at the distal end of a lancing device, such as, for example, an in-situ meter wherein the lancing element 15 extends through opening 32 to puncture skin. Milking ring 32 may be, for example, a substrate with a hole drilled through it which could be, for example, a plastic such as polystyrene, polyethylene, polycarbonate, polyester, or the like. The diameter of opening 32 of said milking ring may be, for example, in the range of between 3.5 and 12 mm. In operation, the milking ring 31 may be applied with gentle pressure onto a fingertip, forearm, or other suitable site such that the skin surface 30 forms a raised mound within milking ring 31. In a one embodiment according to the present invention, milking ring 31 is applied to skin surface 31 with a pressure of approximately 0.5 to 1.5 pounds of applied pressure. In one embodiment according to the present invention, the use of a milking ring is intended to facilitate the collection of bodily fluids by applying a pressure around the incision site to provide a driving force for expressing fluid from the wound site.

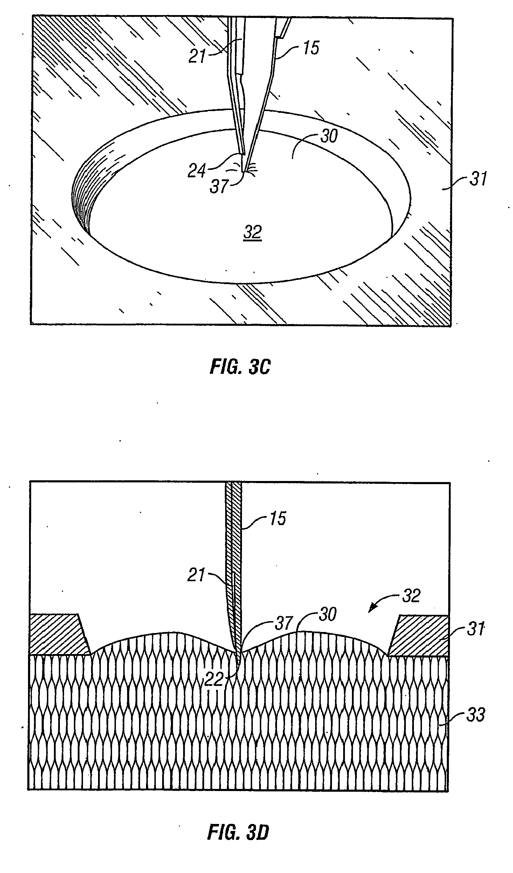

Figure 3C is a perspective view of a lancing element as the tip of the lancing element begins to penetrate the surface of the skin. As lancing tip 22 enters skin surface 30, deflecting skin surface 30 away from lancing tip 22 until skin surface 30 is punctured, forming an incision 37 in skin surface 30, enabling lancing element 15 to enter subcutaneous region 33. Figure 3D is a side view of the lancing element as the tip of the lancing element begins to penetrate the surface of the skin. More particularly, Figure 3D is a side view of lancing element 15 as lancing tip 22 enters incision 37 in skin surface 30.

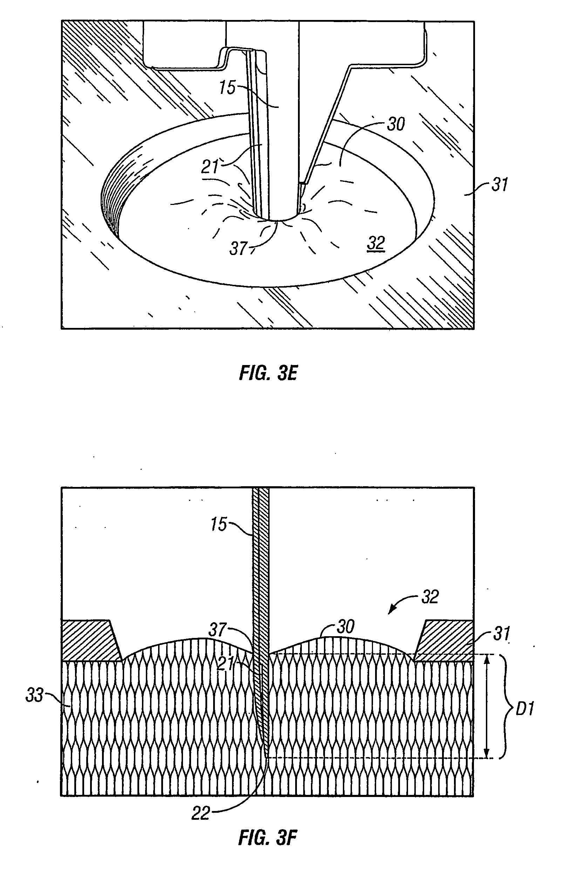

Figure 3E is a perspective view of the lancing element as it reaches its full depth of penetration beneath the surface of the skin. In one embodiment of the present invention, the penetration depth may be reached by extending the lancing tip 22 to a first extension length beyond opening 32. More particularly, Figure 3E is a perspective view of lancing element 15 after lancing tip 22 has reached its full depth of penetration into subcutaneous region 33. Figure 3F is a side view of the lancing element 15 as it reaches its full depth of penetration beneath the surface of the skin. More particularly, Figure 3F is a side view of lancing element 15 after lancing tip 22 has reached its full depth of penetration into subcutaneous region 33. At full penetration, lancing tip 22 reaches a depth of D1. The actual value of D1 for a particular application will depend upon a number of factors, including the bodily fluid being extracted. For example, if the bodily fluid being extracted is blood, the depth D1 will be greater than if the bodily fluid being extracted is interstitial fluid (i.e. ISF). In one embodiment of the present invention, penetration depth D1 may be, for example, in the range of 0.25 to 1.5 mm deep. In a further embodiment of the present invention, D1 may represent the first extension length measured from lancing tip 22 to opening 32.

Insertion of lancing element 15 through skin surface 30 creates an incision 37 in addition to severing subcutaneous tissue and capillaries and providing fill channel 21 with access to the bodily fluid to be sampled, whether blood or interstitial fluid. Thus, with lancing element 15 positioned as shown in Figures 3E and 3F, bodily fluid will flow through fill channel 21 and into sensor strip 100. However, leaving lancing element 15 positioned as illustrated in Figures 3E and 3F will not provide an optimal flow of bodily fluid through fill channel 21. The reasons for the limited flow may include, for example, the blocking of lanced capillaries by the location of lancing element 15 which may, for example, prevent the capillaries or interstitial fluid from flowing freely and pooling in the wound created by lancing element 15.

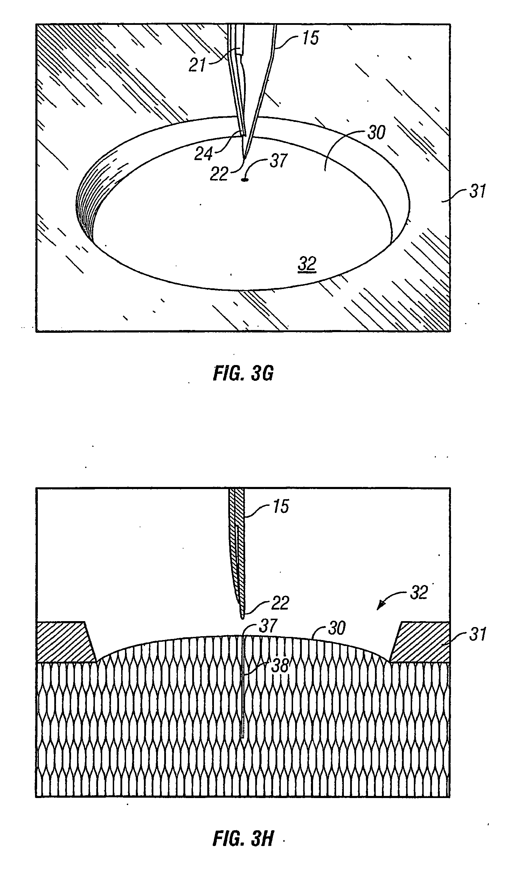

Fig 3G is a perspective view of the lancing element after it is fully withdrawn from the skin. More particularly, Figure 3G is a perspective view of lancing element 15 after lancing tip 22 has been withdrawn completely from incision 37. Fig 3H is a side view of the lancing element 15 after it is fully withdrawn from skin surface 30. In a further embodiment of the present invention, lancing tip 22 is withdrawn fully past opening 32.
Fully withdrawing lancing element 15 from incision 37 creates an open wound 38 below incision 37 that facilitates expression of bodily fluid into wound 38. By fully removing lancing element 15 from the wound
bodily fluids flow more readily into wound 38. If lancing element 15 is not completely removed from wound 38, blood and/or interstitial fluid flow may be impeded. One possible explanation for the limited blood flow is the possibility that the partially retracted lancing element 15 may still effectively block severed capillaries because of the resiliency of the skin. After the initial skin stretching during the penetration event, the skin might revert back to its initial position around lancing element 15. Thus, in
it is important that lancing element 15 be fully removed from wound 38 after the initial penetration to allow bodily fluid to pool in wound 38.

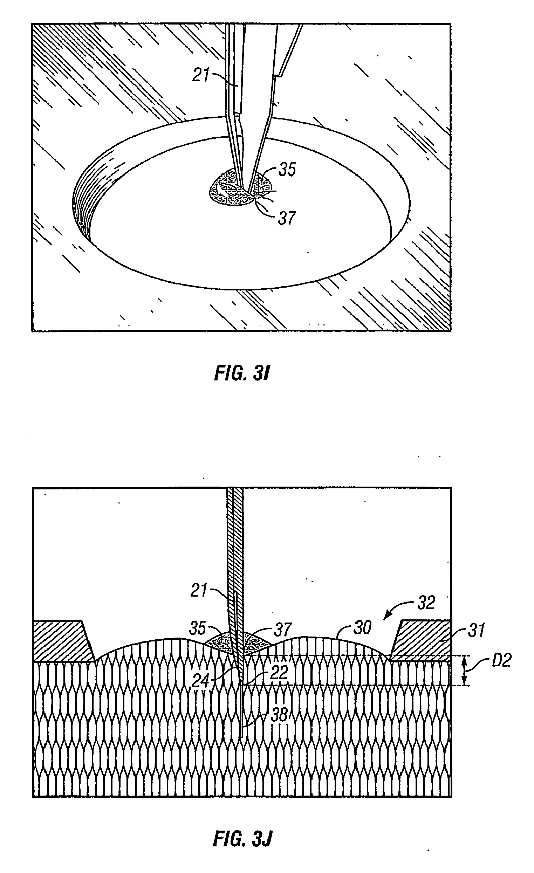

Figure 3I is a perspective view of the lancing element as it re-enters the surface of the skin through the incision created during the first entry. More particularly, Figure 3I is a perspective view of lancing element 15 after lancing tip 22 has been re-inserted through incision 37 into wound 38. Figure 3J is a side view of the lancing element as it re-enters the surface of the skin through the incision created during the first entry. More particularly, Figure 3J is a side view of lancing element 15 positioned within wound 38 such that channel tip 24 is below skin surface 30. Illustrated in Figure 3J, lancing tip 22 is reinserted to a depth D2 which is less than D1. In a further embodiment of the present invention, D2 may represent a second extension length, wherein lancing tip 22 is extended past opening 32 to second extension length D2, in this embodiment of the invention, wherein second extension length D2 is less than first extension length D1. Partially reinserting facilitates the flow of bodily fluids into fluid channel 21. In one embodiment of the present invention, the distance D2 to which lancing tip 22 penetrates is generally defined by the distance from lancing tip 22 to channel tip 22. In one embodiment of the invention, lancing tip 22 may be reinserted to a depth D2 of approximately 0.05 to 0.25 mm deep, wherein D2 is defined as the distance from a surface of the skin 34 to the lancing tip 22.

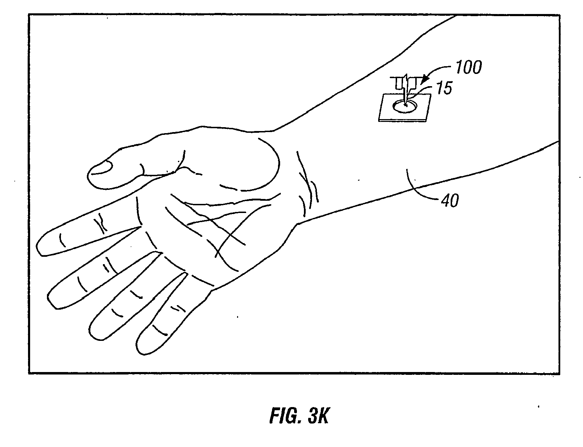

Figure 3K is a perspective view showing the lancing element being used to draw blood from a forearm. More particularly, Figure 3K is a perspective view of a lancing device 15 being used, to draw bodily fluids from a forearm 40 of a human being.

In a method of lancing skin , as set forth above, the method may further include using milking ring 31 to exert the pressure in the region surrounding incision 37 exerted by milking ring 31. Milking ring 31 is positioned on the skin prior to the step of forcing the lancing tip 22 into the skin and may be maintained throughout the remainder of the procedure. In this embodiment of the invention, the milking ring 31 provides a pressure sufficient to facilitate the flow of bodily fluids into fill channel 21 after the reinsertion of lancing tip 22 into wound 38. In one embodiment of the invention, milking ring 31 provides a pressure in a range of approximately 0.5 to 1.5 pounds.
The method may include positioning the milking ring 31 against the skin for a predetermined period of time prior to launching the lancing element. The predetermined period of time may be three seconds or more.

It will be recognized that equivalent structures may be substituted for the structures illustrated and described herein and that the described embodiment of the invention is not the only structure which may be employed to implement the claimed invention. As one example of an equivalent structure which may be used to implement the present invention, a lancing element may be used which does not include a channel tip, with the channel extending from the distal end of the lancing element to the working electrode. While preferred embodiments of the present invention have been shown and described herein, it will be obvious to those skilled in the art that such embodiments are provided by way of example only. Numerous variations, changes, and substitutions will now occur to hose skilled in the art without departing from the invention. It should be understood that various alternatives to the embodiments of the invention described herein may be employed in practicing the invention. It is intended that the following claims define the scope of the invention and that methods and structures within the scope of these claims and their equivalents be covered thereby.

## Claims

1. A lancing instrument for lancing skin, the lancing instrument having proximal and distal ends and comprising:
a lancing element having a sharpened end;
a fluid channel extending from the sharpened end;
a channel tip integrated with the lancing element at the distal end and offset in the proximal direction from the sharpened end of the lancing element; wherein:
said lancing element is adapted for insertion to form an incision;
said channel tip is adapted to increase the separation of the incision; and
said lancing element and said channel tip are adapted to be maintained in said incision while bodily fluids are drawn into the fluid channel.

2. The lancing instrument of claim 1, further comprising a sensor strip attached to a proximal portion of said lancing element.

3. The lancing instrument of claim 2, further adapted such that said bodily fluids are drawn through the fluid channel to said sensor strip.

4. The lancing instrument of claim 2 or claim 3, wherein the channel extends to the sensor strip.

5. The lancing instrument of any preceding claim, wherein the sharpened end of the lancing element may be inserted into a patient's skin to a first predetermined depth.

6. The lancing instrument of any preceding claim, wherein the sharpened end of the lancing element may be inserted into a patient's skin to a second predetermined depth which is not as deep as the first predetermined depth, to enable bodily fluids to be drawn in to the fluid channel.

7. The lancing instrument of claim 5 or 6, wherein the first predetermined depth is between 0.25mm and 1.5mm.

8. The lancing instrument of claim 6, or claim 7 when dependent on claim 6, wherein the second predetermined depth is between 0.05mm and 0.25mm.

9. The lancing instrument of any preceding claim, wherein the channel tip is offset from the sharpened end of the lancing element by between about 0.12mm and about 0.51mm.
